# EUROPEAN PATENT APPLICATION

(11) **EP 4 245 389 A1**
(43) Date of publication of application: **20.09.2023**
(21) Application number: 23160997.5
(22) Date of filing: 09.03.2023
(51) Int. Cl.: A63F 13/65, A61B 5/00, G04G 21/02, A63F 13/212

(54) **GAME INPUT SYSTEM AND METHOD**

(30) Priority: 18.03.2022 GB 202203787
(71) Applicant: Sony Interactive Entertainment Inc., Tokyo 108-0075 (JP)
(72) Inventor: UBERTI, David, London, W1F 7LP (GB); QUIROS, Estefania, London, W1F 7LP (GB); WALKER, Andrew, London, W1F 7LP (GB); EDER, Michael, London, W1F 7LP (GB); MURPHY, Alan, London, W1F 7LP (GB)
(74) Representative: D Young & Co LLP

(57) **Abstract**

An information processing apparatus, comprising: a storage unit configured to store one or more criteria associated with a video game; an executing unit configured to execute the video game; an input unit configured to receive activity data, wherein the activity data is data associated with physical activity of a user who is not directly playing the video game; and a processing unit configured to modify one or more aspects of a virtual environment within the executed video game when the received activity data satisfies one or more of the criteria.

## Description

### Field of Invention

The present invention relates to a game input system and method.

### Background

The popularity of multi-player video games has increased in recent years. Such multi-player video games allow users to connect with other users while completing certain achievements or challenges within the video game. For example, in order to complete certain achievements or challenges within a multi-player video game, two or more users may need to co-operate with each other. For example, the two or more users may need to help each other in order to overcome a certain obstacle or defeat a mutual enemy. In other examples, completing certain achievements or challenges may require the two or more users to compete with each other. For example, the two or more users may be split into two or more teams, and the challenge is to obtain more points, kills, goals, etc. than the other team.

However, multi-player games are typically limited to certain locales. For example, multi-player games may be played locally on one video game console by using two or more video game controllers. In another example, multi-player video games may be played over a network such as LAN, the Internet, or the like, wherein each user plays the video game on their respective video game console. In both examples, users are constrained to play the multi-player video game in a certain locale, this locale corresponding to the connectivity range of a video game controller to a video game console.

The present invention seeks to alleviate or mitigate this issue.

### Summary of the Invention

In a first aspect, an information processing apparatus is provided in claim 1.

In another aspect, an information processing system is provided in claim 6.

In another aspect, an information processing method is provided in claim 11.

Further respective aspects and features of the invention are defined in the appended claims.

Embodiments of the present invention will now be described by way of example with reference to the accompanying drawings, in which:
- Figure 1 schematically illustrates an entertainment system operable as an information processing apparatus according to embodiments of the present description;
- Figure 2 schematically illustrates an information processing apparatus according to embodiments of the present description;
- Figure 3 schematically illustrates an information processing apparatus according to embodiments of the present description;
- Figure 4 schematically illustrates an information processing system according to embodiments of the present description;
- Figure 5 schematically illustrates an information processing system according to embodiments of the present description;
- Figure 6 schematically illustrates an information processing method according to embodiments of the present description; and
- Figure 7 schematically illustrates an information processing method according to embodiments of the present description.

### Description of the Embodiments

An information processing apparatus, system and method are disclosed. In the following description, a number of specific details are presented in order to provide a thorough understanding of the embodiments of the present invention. It will be apparent, however, to a person skilled in the art that these specific details need not be employed to practice the present invention. Conversely, specific details known to the person skilled in the art are omitted for the purposes of clarity where appropriate.

In an example embodiment of the present invention, an entertainment system is a non-limiting example of such an information processing apparatus.

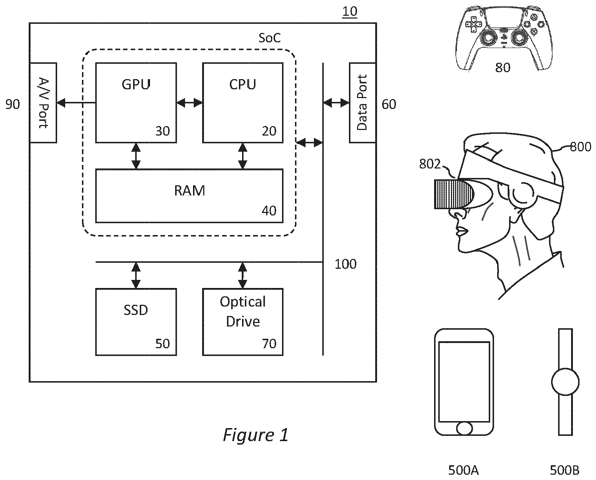

Referring to Figure 1, an example of such an entertainment system 10 is a computer or console such as the Sony ^{®} PlayStation 5 ^{®} (PS5).

The entertainment system 10 comprises a central processor 20. This may be a single or multi core processor, for example comprising eight cores as in the PS5. The entertainment system also comprises a graphical processing unit or GPU 30. The GPU can be physically separate to the CPU, or integrated with the CPU as a system on a chip (SoC) as in the PS5.

The entertainment device also comprises RAM 40, and may either have separate RAM for each of the CPU and GPU, or shared RAM as in the PS5. The or each RAM can be physically separate, or integrated as part of an SoC as in the PS5. Further storage is provided by a disk 50, either as an external or internal hard drive, or as an external solid state drive, or an internal solid state drive as in the PS5.

The entertainment device may transmit or receive data via one or more data ports 60, such as a USB port, Ethernet ^{®} port, WiFi ^{®} port, Bluetooth ^{®} port or similar, as appropriate. It may also optionally receive data via an optical drive 70.

Interaction with the system is typically provided using one or more handheld controllers 80, such as the DualSense ^{®} controller in the case of the PS5.

Audio/visual outputs from the entertainment device are typically provided through one or more A/V ports 90, or through one or more of the wired or wireless data ports 60.

Where components are not integrated, they may be connected as appropriate either by a dedicated data link or via a bus 100.

An example of a device for displaying images output by the entertainment system is a head mounted display 'HMD' 802, worn by a user 800.

In addition a second processing apparatus (500, 900) may operate in conjunction with the first processing apparatus 10 as part of a combined system, with phone 500A and fitness tracker 500B being non-limiting examples of the second processing apparatus (500, 900), as discussed later herein.

As previously mentioned, users playing a video game are often constrained to a certain locale, this locale corresponding to the connectivity range of a video game controller to a video game console. This typically results in users adopting a sitting position while they play the video game. Additionally, users typically play video games for up to several hours at a time. This can result in users being physically inactive for long periods of time, as the sitting position users typically adopt is not conducive to physical exercise. This lack of physical activity can lead to issues with user fitness.

Moreover, video games typically require input from a video game controller, keyboard, mouse, or the like in order to control an in-game character, object, viewpoint or the like. Thus, users often perform input actions similar to that of other users in order to provide an input to the video game, such as pressing a button or key. In the case of multi-player video games, particularly co-operative multi-player video games, this similarity in input actions could result in some users perceiving their contributions to the gameplay as being indistinct from that of other users, leading to a dissatisfaction in playing the video game. For such users, there may be a desire to possess a different mode of providing inputs to a video game console in order to contribute to the gameplay in a different manner to that typically provided to users.

Furthermore, in certain video games, a user may be required to perform certain tasks within the video game in order to unlock a particular item, power-up, consumable or the like for their in-game character. Some users may find these tasks to be too difficult, or repetitive, for example, leading to a dissatisfaction in playing the video game. Previous attempts to overcome this issue often involve providing options for the user to purchase the particular item, power-up, consumable or the like from an in-game marketplace using either in-game or real-world currency. However, some users prefer not to spend additional money. Therefore, it appears that certain video games could benefit from providing an alternative method of enabling users to unlock certain items, power-ups, consumables or the like to users, or otherwise modify aspects of the game.

The aforementioned problems can be mitigated or alleviated by implementing an alternative yet complementary mode of providing inputs to a video game based on physical activity of a user who is not directly playing the video game.

Firstly, this complementary mode of providing inputs would encourage users to engage in physical activity outside of the aforementioned restricted locale, as such physical activity can be used as input for the video games they play. Secondly, this complementary mode of providing inputs would satisfy those users who desire to possess an alternative mode of providing inputs to a video game. Thirdly, this complementary mode of providing inputs would enable users to unlock items, power-ups, consumables or the like without having to endure difficult or repetitive in-game tasks, or purchasing said items, power-ups, consumables or the like.

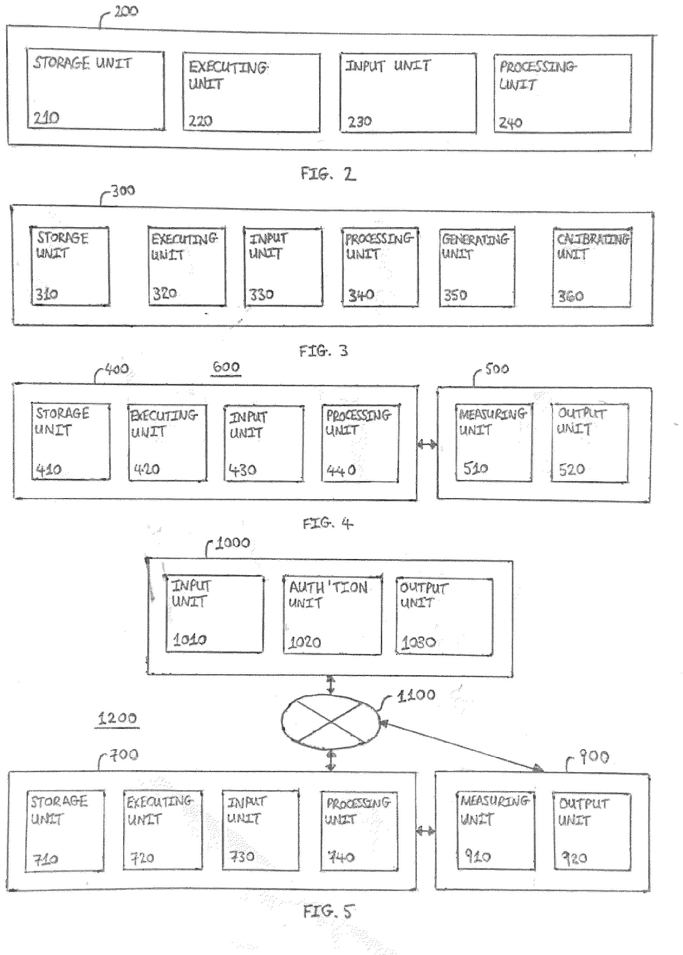

Accordingly, turning now to Figure 2, in embodiments of the present description, an information processing apparatus 200 comprises a storage unit 210 (for example SSD 50 or RAM 40 of entertainment device 10) configured to store one or more criteria associated with a video game; an executing unit 220 (for example CPU 20 of entertainment device 10 operating under suitable software instruction) configured to execute the video game; an input unit 230 (for example data port 60 of entertainment device 10) configured to receive activity data, wherein the activity data is data associated with physical activity of a user who is not directly playing the video game; and a processing unit 240 (again for example CPU 20 of entertainment device 10 operating under suitable software instruction) configured to modify one or more aspects of a virtual environment within the executed video game when the received activity data satisfies one or more of the criteria.

Embodiments of the present description enable users to contribute to the gameplay of a video game by way of performing physical activity which would not normally be considered related to the playing of the video game. For example, a user may be running through a park. Moreover, the user may be wearing, say, a fitness tracking device which measures activity data such as heart rate, calories burnt, a number of steps taken, or the like while the user is running. This activity data, which has been measured while the user is performing physical activity not directly related to the playing of the video game, can be input into the aforementioned information processing apparatus. This information processing apparatus, which may be, say, entertainment device 10 or another computing device capable of executing video games, may subsequently utilise this activity data in order to modify one or more aspects of the virtual environment within the video game. For example, the activity data can be used to complete in-game challenges, levels or the like, or to unlock items, power-ups, consumables or the like.

Moreover, embodiments of the present description may enable users to partake in novel multi-player gaming experiences. For example, a multi-player video game may require one or more users to provide game inputs via video game controllers, keyboards, mice or the like, and one or more other users to provide activity data by performing physical activity not directly related to the video game. The video game may be played co-incidentally with the performance of the physical activity. Consequently, the activity data measured during the physical activity may be live-streamed to the information processing apparatus executing the video game. Alternatively, activity data may be transmitted to the information processing apparatus periodically, such as, say, after every 5, 10, 30, 45 or 60 seconds, or the like.

In embodiments, the multi-player video game may be a co-operative multi-player video game. In such embodiments, the multi-player video game may require a combination of game inputs (provided via video game controllers, keyboards, mice, or the like) and activity data (provided via a fitness tracking device or the like) in order to complete a certain in-game challenge or level, or unlock items, power-ups, consumables, or the like. For example, defeating a boss in a video game may require a combination of game inputs which cause an in-game character to fire a weapon at the boss, and activity data (such as heart rate data) which meets certain criteria (heart rate data must be sustained above, say, 150 bpm for, say, 2 minutes) in order to provide a supply of ammunition for the weapon being fired at the boss.

In other embodiments, the multi-player video game may be a competitive multi-player video game. In such embodiments, the multi-player video game may pit one or more first users providing game inputs to the video game against one or more second users providing activity data to the video game. For example, an in-game challenge may require in-game characters may to remain standing upon a platform for, say, 3 minutes, while the angle of inclination of the platform is varied over time. The one or more first users may be required to control the in-game characters by providing game inputs in order to successfully complete the challenge. For example, the provided game inputs may cause the in-game character to move in a certain direction, jump, crouch, lie prone, crawl, or the like. The one or more second users may be required to adjust the angle of inclination of the platform by providing activity data in order to prevent the successful completion of the in-game challenge. For example, the angle of inclination may be adjusted to, say, 20 degrees when the activity data (such as heart rate data) meets certain criteria (heart rate above 150 bpm).

Alternatively or in addition, embodiments of the present description may enable users to partake in novel single-player experiences. In such embodiments, the single-player game may require a user to unlock a certain in-game item in order to complete an in-game challenge. For example, in order to defeat a boss, the in-game character may be required wield a particular in-game weapon that possesses certain characteristics which allow it to inflict damage on the boss. In order to unlock and wield this particular in-game weapon, the user may be required to run for a distance of, say, 1 mile. Such a requirement may comprise one or more criteria which the activity data must meet. For example, a first criteria may be that the distance travelled must be at least 1 mile (the requisite distance to unlock the in-game weapon), and a second criteria may be that the velocity of travel must be between 5 mph and 20 mph (ensuring that the user does not use a different mode of travel, such as walking, or using a vehicle).

During the run, the fitness tracking device may continuously measure activity data such as the distance that the user has covered while running, and the velocity at which the user is running. Alternatively, the activity data may be measured periodically, such as after every 30, 40 or 50 seconds, for example. The run may end when the user reads the display of the fitness tracking device and notices that they have run the requisite distance. When the user returns to play the video game, the user may transmit the activity data associated with the run from their fitness tracking device to their, say, video game console executing the single-player video game. Alternatively, the activity data may have been transmitted to a server during the run, in which case the user may download the activity data from the server onto the video game console.

A processing unit of the video game console may then determine whether the inputted activity meets the one or more criteria for unlocking the item. For example, the processing unit may determine whether the distance travelled during the run is at least 1 mile, and that the velocity of travel during the run was between 5 mph and 20 mph. Once the activity data has been found to meet the criteria, the in-game weapon may be unlocked for the user.

Notably therefore in each case the game comprises a combination of conventional gaming inputs (for example using a hand held controller) in proximity at least to the display from the information processing apparatus, and also a fitness based activity typically not directly related to playing the video game and free to occur distant from such a display and the information processing apparatus itself; gaming inputs and fitness activities may occur substantially simultaneously where multiple people are playing the game, or may occur sequentially for only one person who is playing the game.

As previously mentioned, in embodiments of the present description, information processing apparatus 200 may be an entertainment device 10 such as a computer, a video game console, or the like.

In embodiments of the present description, storage unit 210 may be a system memory such as RAM 40, ROM, or the like. In other embodiments, storage unit 210 may be a storage memory such as HDD, SSD 50, CD, Floppy Disk, or the like.

In embodiments of the present description, storage unit 210 is configured to store one or more criteria associated with a video game. The one or more criteria associated with a video game may be one or more criteria which must be satisfied by a user in order that one or more aspects of a virtual environment in a video game can be modified. In embodiments of the present description, such criteria may correspond to one or more aspects of a performance of a physical activity. For example, the one or more criteria may correspond to one or more aspects such as a heart rate, a distance travelled, a location reached, a number of steps taken, a velocity, an acceleration, a breathing rate, a number of calories burnt, a detection motion of at least part of a human body, or the like.

A criterion corresponding to a heart rate may be a threshold heart rate which must be achieved by the user, such as 150, 160, or 170 bpm, for example. Moreover, more than one such criterion may be set. For example, a first threshold heart rate may be set as 150, 160, or 170 bpm, and a second threshold heart rate may be set as 170, 180, or 190 bpm. Thus, one or more criteria associated with a video game may comprise one or more threshold heart rates to be achieved by the user. Alternatively or in addition, a criterion corresponding to a heart rate may be a specified minimum period of time during which the user's heart rate must exceed a threshold heart rate. For example, the criterion may be that the user is to maintain their heart rate above 120, 130, or 140 bpm for at least 5, 6, or 7 minutes. Thus, one or more criteria associated with a video game may comprise a minimum period of time during which the heart rate of the user exceeds a threshold heart rate. It will also be appreciated that rather than absolute bpm values, such criteria may be relative, for example 25% or 50% above a previously established resting bpm of the user. As will be appreciated by persons skilled in the relevant art, such criteria should specify heart rates that are reasonably expected for the given user to achieve. A user that is 65 years of age may not reasonably be expected to achieve a heart rate of 170 bpm during physical activity. User-specific criteria shall be discussed later herein.

A criterion corresponding to a distance travelled may be a threshold distance which must be travelled by the user, such as 1, 2, or 3 miles, for example. Moreover, more than one such criterion may be set. For example, a first threshold distance may be set as 1, 2, or 3 miles, and a second threshold distance may be set as 3, 4, or 5 miles. Thus, one or more criteria associated with a video game may comprise one or more threshold distances to be travelled by the user. Distance may also be defined functionally, for example as a number of laps of a standard running track, or a number of lengths of a swimming pool. As will be appreciated by persons skilled in the relevant art, such criteria should specify distances that are reasonably expected for the given user to travel. A user that is 65 years of age may not reasonably be expected to travel 10 miles during physical activity. User-specific criteria shall be discussed later herein.

A criterion corresponding to a location reached may be a set of GPS coordinates to which the user must travel, for example. Alternatively or in addition, such a criterion may be an address, a landmark, or the like. Moreover, more than one such criterion may be set. For example, a first location may be set as 55°51'58" N, 4°16'23.3" W (a location along Sauchiehall Street, Glasgow, UK), and a second location may be set as "Nelson Mandela Place, Glasgow, UK". Thus, one or more criteria associated with a video game may comprise one or more locations to be reached by the user. As will be appreciated by persons skilled in the relevant art, such criteria should specify locations that are reasonably expected for the given user to reach. A user located in Cardiff may not reasonably be expected to reach a location in Glasgow during physical activity. Criteria specific to user location shall be discussed later herein.

A criterion corresponding to a number of steps taken may be a threshold number of steps which must be taken by the user, such as 500, 1,000, or 1,500 steps, for example. Moreover, more than one such criterion may be set. For example, a first threshold number of steps may be set as 500, 1,000, or 1,500 steps, and a second threshold number of steps may be set as 1,500, 2,000, or 2,500 steps. Thus, one or more criteria associated with a video game may comprise one or more threshold numbers of steps to be taken by the user. As will be appreciated by persons skilled in the relevant art, such criteria should specify numbers of steps that are reasonably expected for the given user to take. A user whose mobility is restricted may not reasonably be expected to take 2,500 steps during physical activity. User-specific criteria shall be discussed later herein.

A criterion corresponding to a velocity may be a threshold velocity which must be achieved by the user, such as 7, 8, or 9 mph. Moreover, more than one such criterion may be set. For example, a first threshold velocity may be set as 7, 8, or 9 mph, and a second threshold velocity may be set as 9, 10, or 11 mph. Thus, one or more criteria associated with a video game may comprise one or more threshold velocities to be achieved by the user. Alternatively or in addition, a criterion corresponding to a velocity may be a specified minimum period of time during which the user's velocity must exceed a threshold velocity. For example, the criterion may be that the user is to maintain their velocity above 10, 11, or 12 mph for at least 5, 6, or 7 minutes. Thus, one or more criteria associated with a video game may comprise a minimum period of time during which the velocity of the user exceeds a threshold velocity. As will be appreciated by persons skilled in the relevant art, such criteria should specify velocities that are reasonably expected for the given user to achieve. A user whose mobility is restricted may not reasonably be expected to run at 10 mph during physical activity. User-specific criteria shall be discussed later herein.

A criterion corresponding to an acceleration may be a threshold period of time in which the user must achieve a threshold velocity. For example, a threshold period of time may be set as 3, 4, or 5 seconds, and a threshold velocity may be set as 12, 13 or 14 mph. In this example, the user may either be travelling at a relatively lower velocity, such as 7, 8, or 9 mph, or be standing still. In either case, the user must accelerate such that they can achieve the threshold velocity of 12, 13 or 14 mph in under the specified 3, 4 or 5 seconds. Thus, one or more criteria associated with a video game may comprise a threshold velocity to be achieved by the user within a threshold period of time. As will be appreciated by persons skilled in the relevant art, such criteria should specify velocities and periods of time that are reasonably expected for the given user to achieve. A user whose mobility is restricted may not reasonably be expected to achieve 14 mph from a standing start in under 4 seconds during physical activity. User-specific criteria shall be discussed later herein.

A criterion corresponding to a breathing rate may be a threshold breathing rate which must be achieved by the user, such as 35, 40 or 45 breaths per minute, for example. Moreover, more than one such criterion may be set. For example, a first threshold breathing rate may be set as 35, 40, or 45 breaths per minute, and a second threshold heart rate may be set as 50, 55, or 60 breaths per minute. It will also be appreciated that rather than absolute breathing rates, such criteria may be relative, for example 25% or 50% above a previously established resting breathing rate of the user. Thus, one or more criteria associated with a video game may comprise one or more threshold breathing rates to be achieved by the user. Alternatively or in addition, a criterion corresponding to a breathing rate may be a specified minimum period of time during which the user's breathing rate must exceed a threshold breathing rate. For example, the criterion may be that the user is to maintain their breathing rate above 40, 45, or 50 breaths per minute, for at least 5, 6, or 7 minutes. Thus, one or more criteria associated with a video game may comprise a minimum period of time during which the breathing rate of the user exceeds a threshold breathing rate. As will be appreciated by persons skilled in the relevant art, such criteria should specify breathing rates that are reasonably expected for the given user to achieve. A user that is 65 years of age may not reasonably be expected to achieve a breathing rate of 55 breaths per minute during physical activity. User-specific criteria shall be discussed later herein.

A criterion corresponding to a number of calories burnt may be a threshold number of calories which must be burnt by the user, such as 100, 200, or 300 calories, for example. Moreover, more than one such criterion may be set. For example, a first threshold number of calories may be set as 100, 200, or 300 calories, and a second threshold number of steps may be set as 300, 400 or 500 calories. Thus, one or more criteria associated with a video game may comprise one or more threshold numbers of calories to be burnt by the user. As will be appreciated by persons skilled in the relevant art, such criteria should specify numbers of calories that are reasonably expected for the given user to burn. A user whose mobility is restricted may not reasonably be expected to burn 500 calories during physical activity. User-specific criteria shall be discussed later herein.

A criterion corresponding to a detected motion of at least part of a human body may be a threshold number of repetitions for which at least part of the user's body must perform a predetermined motion. For example, a threshold number of repetitions may be set as 12, 13, or 14 repetitions, and the part of the user's body may be specified as, say, the user's right arm. Continuing with this example, the predetermined motion may be a motion that part of the user's body must perform, such as a bicep curl, a tricep extension, or the like. The user's motion may be detected by, say, an accelerometer of a fitness tracking device being worn by the user, for example. Detecting user motion shall be discussed later herein. Moreover, repetitions are often performed in one or more sets. For example, a user may perform 3 sets of bicep curls, where each set comprises 12 repetitions. Thus, one or more criteria associated with a video game may comprise a threshold number of sets for which at least part of the user's body must perform a predetermined motion, wherein each set comprises one or more repetitions. As will be appreciated by persons skilled in the relevant art, such criteria should specify numbers of sets and/or repetitions that are reasonably expected for the given user to perform. A user whose mobility is restricted may not reasonably be expected to perform bicep curls for 3 sets of 12 repetitions during physical activity. User-specific criteria shall be discussed later herein.

For some of the aforementioned criteria, an additional threshold defining an upper limit for what is reasonably expected for any user performing physical exercise may be set. For example, an upper limit threshold velocity may be set as 19, 20, or 21 mph. Setting such an upper limit threshold would ensure that the user, regardless of age, mobility, or the like, is not required to run faster than what is reasonably expected of them. This would ensure that the user does not injure themselves in attempting to achieve an unrealistically high threshold velocity while running, such as 25, 26, or 27 mph, for example. Thus, one or more criteria associated with a video game may comprise one or more thresholds which define an upper limit on an expected performance of physical activity.

Hence for any suitable physical activity, the thresholds may be limited to reasonable and safe bounds, and these bounds and/or the criteria within them may be adjusted for the demographics, physical ability, fitness, or the like, of the user.

In embodiments of the present description, executing unit 220 may be one or more CPUs 20, one or more GPUs 30, or any combination of the preceding, for example.

In embodiments of the present description, executing unit 220 is configured to execute a video game. Executing a video game typically involves executing at least part of a code of the video game in order to launch, maintain, or modify/update the video game in a state of play. A state of play may be any state of the video game wherein inputs are required from the user in order to modify the state. For example, a state of play may be an in-game menu, a viewpoint of an in-game virtual environment, the location and/or orientation of one or more objects within the in-game virtual environment, the location and/or orientation of one or more characters within the in-game virtual environment, or the like. As will be appreciated by persons skilled in the relevant art, modifying the state of play (e.g., selecting an option in the in-game menu, changing the position and/or orientation of the viewpoint, changing the location and/or orientation of one or more of the objects, changing the location and/or orientation of one or more of the characters) requires an input to be provided by the user. Thus, executing a video game comprises executing at least a part of a code of the video game in order to launch, maintain, or modify/update the video game in a state of play.

In embodiments of the present description, input unit 230 may be one or more data ports, such as USB ports, Ethernet ^{®} ports, WiFi ^{®} ports, Bluetooth ^{®} ports, or the like.

In embodiments of the present description, input unit 230 is configured to receive activity data. Activity data is data associated with physical activity of a user who is not directly playing a video game. The activity data may be data corresponding to one or more aspects of a performance of a physical activity, such as a measured heart rate, velocity, distance travelled, or the like. Types of activity data shall be discussed later herein. The activity data may be measured by using, say, a fitness tracking device, a mobile phone, or the like, which may be worn, held or affixed to the user performing the physical activity. Measuring activity data shall be discussed later herein. A user who is not directly playing a video game may be a user whose performance of a physical activity is such that it precludes them from playing a video game on their video game console while performing the physical activity. For example, a user who is running through a park is precluded from playing a video game on their video game console while running by virtue of being away from their video game console, which may for example still be at home. As will be appreciated by persons skilled in the relevant art, activity data may, therefore, be data corresponding to one or more aspects of a performance of a physical activity by a user, wherein the performance of the physical activity is such that it precludes the user from playing a video game while performing the physical activity.

As noted elsewhere herein in embodiments of the present description, processing unit 240 may be one or more CPUs 20, one or more GPUs 30, or any combination of the preceding, for example.

In embodiments of the present description, processing unit 240 is configured to modify one or more aspects of a virtual environment within an executed video game when activity data satisfies one or more of the criteria (e.g. those selected for that virtual environment). One or more aspects of a virtual environment within the video game may be one or more of: a location and/or orientation of an in-game object, a state of an in-game object, a provision of one or more in-game objects for an in-game character, a location and/or orientation of an in-game character, a state of an in-game character, a number of consumables for an in-game character, a number of power-ups for an in-game character, one or more abilities of an in-game character, a provision of one or more abilities for an in-game character, a difficulty of an in-game challenge, an amount of in-game currency, or the like, for example.

A location and/or orientation of an in-game object may be a location and/or orientation in which the in-game object is placed within the virtual environment. For example, the in-game object may be a mounted telescope. This mounted telescope may be located within a room of a house within the virtual environment. Moreover, this mounted telescope may be oriented such that it provides a view through a window of the room for the user, this view comprising an enemy fortification in the distance. The location and/or orientation of this mounted telescope may be modified. For example, the mounted telescope may be moved to a new location, such as a different room of the house, within a field, on a mountain, along a river, within a settlement, or the like. Alternatively or in addition, the mounted telescope may be oriented such that it provides a view of, say, a field, a mountain, a river, a settlement, or the like. Thus, one or more aspects of a virtual environment within an executed video game may comprise one or more of: a location of an in-game object, and an orientation of an in-game object.

A state of an in-game object may be a state in which an in-game object exists. For example, an in-game object may be a treasure chest. This treasure chest may be in a locked state, which means that the chest may not be openable by the user. The state of this treasure chest may be modified. For example, the state of the treasure chest may be modified such that it may now be in an unlocked state, and may therefore be openable by the user. In another example, the in-game object may be a weapon such as a sword. This sword may be in a damaged state, which means that the sword may not inflict as much damage as it would normally do in its "newly-forged" state. The state of this damaged sword may be modified. For example, the state of the sword may be modified such that it may now be in a "newly-forged" state, and may therefore be capable of inflicting the amount of damage that it would normally do. Alternatively or in addition, the state of this sword may be modified to an "enchanted" state. In this state, the sword may be capable of inflicting a greater amount of damage than it would normally do, or it may provide health to an in-game character who wields the sword, for example. Thus, one or more aspects of a virtual environment within an executed video game may comprise a state in which an in-game object exists.

A provision of one or more in-game objects for an in-game character may be one or more in-game objects which are provided to an in-game character. For example, such an in-game object may be a helmet. Typically, an in-game object is provided to an in-game character once one or more conditions have been satisfied by the in-game character. For example, in order to obtain the helmet, the in-game character may have to complete an in-game challenge, attain a requisite XP level, possess sufficient in-game currency to purchase the helmet from an in-game marketplace, possess sufficiently valuable in-game objects to barter with another in-game character for the helmet, or the like. This provision may be modified. For example, the provision of an in-game object for an in-game character may be modified such that fewer conditions must be satisfied by the in-game character in order to obtain the in-game object. For example, the provision of a helmet may originally be dependent upon the in-game character completing 5 separate in-game challenges, such as killing a number of enemies, destroying a number of enemy vehicles, destroying a number of enemy fortifications, collecting an object from a certain location within the virtual environment, or the like. This provision may be modified such that the provision may now be dependent upon the in-game character completing 1 in-game challenge. Moreover, this provision may be modified such that the provision may now no longer be dependent upon the in-game character satisfying a condition. In this case, the in-game character may be provided with the helmet without being required to complete an in-game challenge, attain a requisite XP level, possess sufficient in-game currency to, possess sufficiently valuable in-game objects, or the like. Alternatively or in addition, the provision of an in-game object for an in-game character may be modified such that one or more of the conditions have been modified, for example. For example, the provision of a helmet may originally be dependent upon the in-game character killing 5 enemies. This provision may be modified such that the provision of a helmet may now be dependent upon the in-game character killing 1 enemy. Thus, one or more aspects of a virtual environment within an executed video game may comprise a provision of one or more in-game objects for an in-game character. Moreover, the provision of one or more in-game objects for an in-game character may be dependent upon the in-game character satisfying a number of conditions. Consequently, modifying the provision of one or more in-game objects for an in-game character may comprise one or more of: modifying the number of conditions, and modifying one or more of the conditions.

A location and/or orientation of an in-game character may be a location and/or orientation in which the in-game character is placed within the virtual environment. For example, the in-game character may be the user's in-game character, that is, an in-game character that the user can control by using, say, a video game controller. This in-game character may be located within a room of a house within the virtual environment. Moreover, this in-game character may be oriented such that the in-game character appears to look through a window in the room. The location and/or orientation of this in-game character may be modified. For example, the in-game character may be moved to a new location, such as a different room of the house, within a field, on a mountain, along a river, within a settlement, or the like. Alternatively or in addition, the in-game character may be oriented such that it appears to look upwards, downwards, straight-ahead, left, right, or the like, or may be oriented such that the body of the in-game character may be exhibiting a standing pose, a crouching pose, a resting pose, or the like. Thus, one or more aspects of a virtual environment within an executed video game may comprise one or more of: a location of an in-game character, and an orientation of an in-game character.

A state of an in-game character may be a state in which an in-game character exists. For example, an in-game character may be in a wounded state, which means that the in-game character may have a low health level, such as, say, 7% health. Alternatively or in addition, the in-game character may have a health level which is continually depleting, such as, say, a 1% reduction in health every 30 seconds. The state of this in-game character may be modified. For example, the health level of the in-game character may be modified such that it may now be at 80, 90, or 100% health. Alternatively or in addition, the in-game character may have, say, a stamina level, a magic level, and/or an armour level, which respectively relate to an amount of stamina, magical power, and/or armour the in-game character currently possesses. These levels may be modified in a similar manner to that described with respect to the health level. Alternatively or in addition, the in-game character may have an experience level (a so-called "XP level"), which relates to an amount of experience points (so-called "XP") the in-game character currently possesses. This experience level may be modified. For example, the experience level may be increased by a certain amount, such as, say, a 1,000 XP. Thus, one or more aspects of a virtual environment within an executed video game may comprise a state in which an in-game character exists. Moreover, a state in which an in-game character exists may comprise one or more of: a health level, a stamina level, a magic level, an armour level, and an experience level.

A number of consumables for an in-game character may be a number of in-game objects which may be consumed by an in-game character. For example, such an in-game object may be an item of food, such as a loaf of bread, a piece of meat, a piece of fish, a piece of cheese, a fruit, a vegetable, or the like. Such items of food may be consumed by the in-game character in order to boost or replenish their health level or stamina level, for example. Alternatively or in addition, an in-game object which may be consumed by an in-game character may be a potion. This potion may be consumed by the in-game character in order to boost or replenish their health level, stamina level, magic level, armour level, or experience level, for example. Moreover, this potion may be used by the in-game character to enchant or reinforce weapons, for example. Alternatively or in addition, an in-game object which may be consumed by the user may be a raw material, such as a quantity of metal ore, a piece of wood, a piece of clay, or the like. These raw materials may be used by the in-game character to create weapons, buildings, containers for food or drink, or the like, for example. Alternatively or in addition, an in-game object which may be consumed by the user may be an amount of ammunition. This ammunition may be used by the in-game character to load a firearm that they may possess, for example. This number of consumables may be modified. For example, there may originally be 2 pieces of meat which may be consumed by the in-game character. This number of pieces of meat may be increased to, say, 5 pieces of meat. Thus, one or more aspects of a virtual environment within an executed video game may comprise a number of in-game objects which may be consumed by an in-game character.

A number of power-ups for an in-game character may be a number of temporary upgrades which, when activated, affect an ability of an in-game character. For example, such a temporary upgrade may be a speed boost which, when activated, enables the in-game character to run faster for a period of time, such as, say, 30 seconds. Alternatively or in addition, such a temporary upgrade may be a jump boost, a damage boost, or the like. These temporary upgrades may, when activated, respectively enable the in-game character to jump higher, inflict more damage, or the like for a period of time. Alternatively of in addition, a number of power-ups for an in-game character may be a number of temporary upgrades which, when activated, affect the state in which the in-game character exists. For example, such a temporary upgrade may be a health boost, a stamina boost, a magic boost, an armour boost, an XP multiplier, or the like. These temporary upgrades may, when activated, respectively enable the in-game character to increase their health level, increase their stamina level, increase their magic level, increase their armour level, or increase the amount of XP earned by multiplying the XP earned by a certain factor for a period of time. This number of power-ups may be modified. For example, there may originally be 2 speed boosts. This number of pieces of meat may be increased to, say, 5 speed boosts. Thus, one or more aspects of a virtual within an executed video game may comprise a number of temporary upgrades which, when activated, affect one or more of: an ability of an in-game character, and a state in which the in-game character exists.

One or more abilities of an in-game character may be one or more actions which are performable by an in-game character, such as walking, running, jumping, casting magical spells, performing a "special attack" which is capable of inflicting significantly higher damage than what is normally inflicted by the in-game character, and the like. This ability may be modified. For example, the running ability may be modified such that it depletes the stamina level of the in-game character at a lower rate than that prior to modification. For example, the running ability may have depleted the stamina level at 5% every 10 seconds, yet after modification, the running ability may now deplete the stamina level at, say, 2% every 10 seconds. Thus one or more aspects of a virtual environment within an executed video game may comprise one or more actions which are performable by an in-game character.

A provision of one or more abilities for an in-game character may be one or more abilities which are provided to an in-game character. For example, such an ability may be that of flight. Typically, an ability is provided to an in-game character once one or more conditions have been satisfied by the in-game character. For example, in order to obtain the ability to fly, the in-game character may have to complete an in-game challenge, attain a requisite XP level, possess sufficient in-game currency to purchase the flying ability from an in-game marketplace, possess sufficiently valuable in-game objects to barter with another in-game character for the flying ability, or the like. This provision may be modified. For example, the provision of an ability for an in-game character may be modified such that fewer conditions must be satisfied by the in-game character in order to obtain the ability. For example, the provision of a flying ability may originally be dependent upon the in-game character completing 5 separate in-game challenges, such as killing a number of enemies, destroying a number of enemy vehicles, destroying a number of enemy fortifications, collecting an object from a certain location within the virtual environment, or the like. This provision may be modified such that the provision may now be dependent upon the in-game character completing 1 in-game challenge. Moreover, this provision may be modified such that the provision may now no longer be dependent upon the in-game character satisfying a condition. In this case, the in-game character may be provided with the helmet without being required to complete an in-game challenge, attain a requisite XP level, possess sufficient in-game currency to, possess sufficiently valuable in-game objects, or the like. Alternatively or in addition, the provision of an in-game object for an in-game character may be modified such that one or more of the conditions have been modified, for example. For example, the provision of a flying ability may originally be dependent upon the in-game character killing 5 enemies. This provision may be modified such that the provision of a flying ability may now be dependent upon the in-game character killing 1 enemy. Thus, one or more aspects of a virtual environment within an executed video game may comprise a provision of one or more in-game objects for an in-game character. Moreover, the provision of one or more in-game objects for an in-game character may be dependent upon the in-game character satisfying a number of conditions. Consequently, modifying the provision of one or more in-game objects for an in-game character may comprise one or more of: modifying the number of conditions, and modifying one or more of the conditions.

A difficulty of an in-game challenge may be a difficulty experienced by a user while attempting to complete an in-game challenge. For example, an in-game challenge may be a final boss fight, wherein a user may be required to defeat the boss in order to complete the in-game challenge. If the final boss fight is set at "high difficulty", the user may experience a greater difficulty while attempting to defeat the boss than when the final boss fight is set at "low difficulty". This may be because, in the "high difficulty" setting, the abilities of the boss have been significantly improved, for example. For example, the boss may be able to run faster, jump higher, inflicted greater damage, or the like. This may result in the boss being more able to evade damaging blows performed by the user's in-game character, kill the user's in-game character in a shorter period of time, or the like, thereby increasing the difficulty experienced by the user in attempting to defeat the boss. Alternatively or in addition, in the "high difficulty" setting, the state of the boss may have been significantly improved, for example. For example, the boss may have an increased health level, stamina level, magic level, armour level, or the like. This may result in the boss being able to withstand a greater number of damaging blows inflicted by the user's in-game character, run for longer periods of time, cast a greater number of magical spells against the user's in-game character, or the like, thereby increasing the difficulty experienced by the user in attempting to defeat the boss. Alternatively or in addition, in the "high difficulty" setting, the number of bosses in the final boss level may have been increased, for example. For example, there may be 2, 3, or 4 bosses which the user must defeat in order to complete the final boss fight. This may result in a greater frequency of damaging blows inflicted upon the user's in-game character, which in turn may result in killing the user's in-game character in a shorter period of time, thereby increasing the difficulty experienced by the user in attempting to complete the final boss fight. This in-game challenge difficulty may be modified. For example, the difficultly of the final boss fight may be modified such that the abilities of the boss have been lessened. For example, the boss may now run slower, inflicted less damage, or the like. Alternatively or in addition, the state of the boss may have been worsened, for example. For example, the boss may now have a reduced health level, stamina level, magic level, armour level, or the like. Alternatively or in addition, the number of bosses may have been reduced, for example. For example, there may now only be 1 boss for the user to defeat instead of 3. Thus, one or more aspects of a virtual environment within an executed video game may comprise a difficulty experienced by a user while attempting to complete an in-game challenge.

An amount of in-game currency may be an amount of in-game currency which may be used to purchase one or more in-game objects, one or more consumables, one or more power-ups, one or more abilities, or the like, for example. For example, an in-game object such as a shield may be on sale (within, say, an in-game marketplace) for 100 in-game credits. The user may wish to purchase this shield to improve their armour level, for example. Subsequently, the user may spend 100 credits of their, say, 2,000 in-game credits in order to purchase the shield. This amount of in-game currency may be modified. For example, the user may originally have 2,000 in-game credits. This amount of in-game currency may be increased to, say, 5,000 in-game credits. Thus, one or more aspects of a virtual environment within an executed video game may comprise an amount of in-game currency.

As previously mentioned, processing unit 240 is configured to modify one or more aspects of a virtual environment within an executed video game when the received activity data satisfies one or more of the criteria. For example, a user's in-game character currently possesses a low health level, such as, say 10% health. This state of the in-game character may be modified such that it now has 80%, 90%, or 100%, for example. In order for processing unit 240 to carry out such a modification to the health level of the in-game character, the user may be required to provide activity data to processing unit 240 via input unit 230. As previously mentioned, activity data is data associated with physical activity of a user who is not directly playing a video game. This activity data may be measured by, say, the user's fitness tracking device. Measuring activity data shall be discussed later herein. Thus, in order to provide processing unit 240 with activity data, the user must temporarily cease directly playing the video game and perform physical activity (while wearing their fitness tracking device), or must be performing the physical activity whilst one or more other users are playing the game. Continuing with the above example, the user may be notified by the executed video game that in order to boost their in-game character's health level, the user must run for a distance of, say, 1 mile. The user may subsequently put on their, say, fitness tracking device to measure the user's activity data while the user goes running.

Once the user has performed physical activity, the user may transmit the activity data from their fitness tracking device to processing unit 240 via input unit 230. Subsequently, processing unit 240 determines whether the received activity data satisfies one or more of the criteria stored in storage unit 210. As will be appreciated by persons skilled in the relevant art, the one or more of the criteria which must be satisfied relate to the physical activity which the user was required to perform. Continuing with the above example, the one or more of the criteria which must be satisfied may be a threshold distance of, say, 1 mile, a threshold velocity of, say, 5 mph, and an upper limit threshold velocity of, say, 20 mph. The threshold distance criteria corresponds to the distance specified in the notification to the user, the threshold velocity criteria may be used to ensure that the user runs for that distance (rather than walking), and the upper limit threshold criteria may again be used to ensure that the user runs for that distance (rather than using a vehicle). Thus, continuing with the above example, processing unit 240 determines whether the received activity data (which was measured while the user was running) comprises a distance travelled measurement of at least 1 mile, and a velocity travelled measurement between 5 mph and 20 mph.

Once processing unit 240 determines that the received activity data satisfies the one or more of the criteria, processing unit 240 may modify the health level of the user's in-game character such that the health level is now, say, 90% health. Thus, processing unit 240 may be configured to determine whether the received activity data comprises data which satisfies one or more of the criteria, and, if so, modify one or more aspects of the virtual environment within the executed the video game.

In another example, the user may wish to acquire an in-game object such as a sword for their in-game character. Consequently, the user (or a co-player) may navigate to an in-game marketplace. The in-game marketplace may notify the user or co-player that the provision of a sword for the in-game character is dependent upon the user or co-player completing an in-game challenge, such as, say, killing 10 enemies with a bow and arrow. The user may not wish to complete this in-game challenge, as they may find it too difficult, or they may have already completed several similar challenges previously and are therefore reluctant to complete another such in-game challenge. In either case, the provision of this sword may be modified such that the provision is no longer dependent upon the in-game character completing an in-game challenge, or indeed, satisfying any in-game condition. In order for processing unit 240 to carry out such a modification to the provision of a sword for the in-game character, the user may be required to provide activity data to processing unit 240 via input unit 230. Continuing with the above example, the in-game marketplace may notify the user that the sword may be provided to the in-game character if the user were to perform, say, 1,000 steps while walking outside. The user may subsequently put on their, say, fitness tracking device to measure the user's activity data while the user goes walking.

Once the user has performed physical activity, the user may transmit the activity data from their fitness tracking device to processing unit 240 via input unit 230. Subsequently, processing unit 240 determines whether the received activity data satisfies one or more of the criteria stored in storage unit 210. Continuing with the above example, the one or more of the criteria which must be satisfied may be a threshold number of steps taken of 1,000 steps, a distance travelled measurement of at least, say, 220 yards (one eighth of a mile), and an upper limit threshold velocity of say, 5 mph. The threshold number of steps taken criteria corresponds to the number of steps specified in the notification to the user, the threshold distance criteria may be used to ensure that the user is not standing in one place while performing the number of steps, and the upper limit threshold velocity may be used to ensure that the user does not run while performing the number of steps. As will be appreciated by persons skilled in the relevant art, too much exercise in a given period of time may lead to a user injuring themselves. Thus, the upper limit threshold criteria of 5 mph may be used to ensure that the user is not over-exercising. For example, the user may have already performed, say, 5 bouts of moderately strenuous physical activity (running at over 15 mph, running at least 5 miles, burning 1,000 calories, performing 5 sets of 15 bicep curls for each arm, or the like) within a period of, say, 3 hours. Thus, it may be prudent to ensure that the user refrains from injuring themselves while performing the 1,000 steps by discouraging them from running. Thus, continuing with the above example, processing unit 240 determine whether the received activity data (which was measured while the user was walking) comprises a number of steps taken measurement of at least 1,000 steps, a distance measurement of at least 220 yards, and a velocity travelled measurement of less than 5 mph.

Once processing unit 240 determines that the received activity data satisfies the one or more of the criteria, processing unit 240 may modify the provision of the sword such that the provision no longer requires the in-game character to satisfy any conditions. Consequently, the sword is provided to the in-game character. Thus, processing unit 240 may be configured to determine whether the received activity data comprises data which satisfies one or more of the criteria, and, if so, modify one or more aspects of the virtual environment within the executed the video game.

It will be appreciated therefore that for various aspects of a virtual environment, such as object or character placement or behaviour, or accessibility of objects such as treasure or swords, or character statistics such as health or speed, the fitness activity of a user who is not directly interacting with the game may be used as a proxy for in-game activities, or may contribute toward those activities, so that the in-game activities may either be made easier, simpler, or partially or wholly skipped, either by the user themselves when subsequently playing the game, or by a co-player playing whilst the user performs the fitness activity. Indeed, where the fitness activity is chosen to be a mandatory part of a game in order to modify a particular aspect of a virtual environment, an alternative in-game activity may be omitted altogether.

Hence as noted elsewhere herein, in embodiments of the present description, the activity data may comprise data such as a heart rate, a distance travelled, a location reached, a number of steps taken, a velocity, an acceleration, a breathing rate, a number of calories burned, and a detected motion of at least part of the user. As will be appreciated by person skilled in the relevant art, activity data is data associated with physical activity of a user who is not directly playing the video game (such a user being previously discussed herein), and is data which must satisfy one or more of the criteria (which were previously discussed herein) in order that one or more aspects of a virtual environment in a video game can be modified.

Turning now to Figure 3, in which parts 310-340 correspond to parts 210-240 of Figure 2 except where stated otherwise, in embodiments of the present description information processing apparatus 300 may comprise generating unit 350 (e.g. CPU 20 of the entertainment device 10 operating under suitable software instruction). Generating unit 350 may be configured to generate, based on data associated with an environment in which physical activity is to be performed, one or more criteria associated with the video game, wherein input unit 330 is configured to receive the data associated with the environment in which physical activity is to be performed. Such embodiments enable the generation of location-specific criteria which are associated with a video game, such criteria being appropriate for the location specified by the user.

For example, a user may be wearing, say, a fitness tracking device while they are currently inside of their, say, house, apartment, accommodation, or the like. The fitness tracking device may be tracking its own the GPS coordinates, and may therefore be tracking the GPS coordinates of the user. The user may transmit this location data (which specifies the location of the user) from the fitness tracking device to input unit 330. Subsequently, generating unit 350 may generate one or more criteria associated with a video game based on this received location data. For example, generating unit 350 may generate a criterion corresponding to a location which must be reached by the user, this generated criterion corresponding to a location that is reasonably expected for the given user to reach. For example, such a criterion may corresponding to a set of GPS coordinates, an address, a landmark, or the like which is, say, no more than 10 miles away from the received location data. Alternatively or in addition, generating unit 350 may generate a criterion corresponding to a specified route through which the user must travel. For example, the location data (which specifies the location of the user) may correspond to, say, a city which is particularly hilly. In such case, generating unit 350 may generate a criterion which specifies a route for the user to travel along which includes, say, a minimal amount of travelling up or down hills. Alternatively or in addition, the user may wish to specify a location in which they would like to perform physical activity, such as a park, gym, football pitch, tennis court, or the like. In such case, the user may input their desired location to input unit 330 via their, say, fitness tracking device, mobile phone, video game controller, or the like. Subsequently, generating unit 350 may generate one or more criteria associated with a video game which corresponds to physical activity to be performed at the specified location. For example, the user may have specified the location of a football pitch. In response, generating unit 350 may generate a criterion corresponding to a heart rate to be achieved at the specified location, such as 150 bpm. The user subsequently goes to play football at the specified location and aims to achieve a heart rate of 150 bpm. Thus, data associated with an environment in which physical activity is to be performed may comprise a specified location. Moreover, one or more criteria generated based on data associated with an environment in which physical activity is to be performed may comprise one or more of: a criterion corresponding to a location which must be reached by the user, wherein the location that is no more than a threshold distance away from the specified location, a criterion corresponding to a specified route through which the user must travel, and a criterion corresponding to physical activity to be performed at the specified location.

In embodiments of the present description, information processing apparatus 300 may comprise calibration unit 360. Calibration unit 360 may be configured to adjust, based on data associated with a current fitness level of the user, one or more of the criteria associated with the video game, wherein the input unit is configured to receive the data associated with a current fitness level of the user. Such embodiments enable the generation of user-specific criteria which are associated with a video game, such criteria being appropriate for the user's current fitness level.

For example, before a notional "operational" phase, where one or more aspects of a virtual environment are modified when the user's activity data satisfies one or more criteria, there may be a "calibration" phase, where the user is required to provide activity data which is representative of their current fitness level. For example, as part of this "calibration" phase, the user may be required to run for, say, half of a mile. The user subsequently puts on their, say, fitness tracking device and goes running. During the run, the fitness tracking device may track, say, the time taken to run the required distance, the user's velocity of travel, the user's heart rate, the number of calories burnt, or the like. This tracked activity data represents the user's current fitness level. When the user transmits this tracked activity data to calibration unit 360 via input unit 330, calibration unit 360 may adjust one or more of the criteria based on this tracked activity data. For example, the maximum heart rate achieved by the user while running may have been, say, 150 bpm. Calibration unit 360 may adjust criteria corresponding to a heart rate, which may have been, say, achieve 170 bpm, maintain heart rate above 150 bpm for 5 minutes, or the like such that the criteria now are, say, achieve 130 bpm, maintain heart above 140 bpm for 3 minutes, or the like. Moreover, there may have been an upper limit threshold heart rate of, say, 180 bpm. Calibration unit 360 may adjust this upper limit threshold heart rate such that it now is, say, 145 or 150 bpm. Alternatively or in addition, the user may be required to answer an initial questionnaire regarding their current fitness level before the "operational" phase begins. For example, calibration unit 360 may output a questionnaire for display to the user, and the user may input their answers to input unit 330 via their, say, fitness tracking device, mobile phone, video game controller, or the like. Subsequently, calibration unit 360 may adjust one or more of the criteria based on these received answers. Thus, data associated with a current fitness level of the user may comprise one or more of: activity data that is representative of the user's current fitness level, and one or more answers to a questionnaire.

In embodiments of the present description, the processing unit (such as processing unit 240, 340 in figures 2 and 3, for example as CPU 20 in Figure 1 under suitable software instruction) may be configured to modify one or more aspects of the virtual environment within the video game based on one or more game inputs. Moreover, input unit 230, 330 may be configured to receive the one or more game inputs. These game inputs may be received from one or more of: a video game controller, a keyboard, a mouse, a microphone, and a camera. Such embodiments enable a novel multi-player gaming experience.

For example, a co-operative multi-player video game may require one or more users to provide game inputs via video game controllers, keyboards, mice or the like, and one or more other users to provide activity data by performing physical activity not directly related to the video game. A first user may, for example, provide one or game inputs such as manipulating a joystick and pressing a button on a video game controller. This game input may, for example, modify an aspect of the virtual environment within the video game, such as causing the first user's in-game character to run in a certain direction. A second user may, for example, provide activity data by wearing a fitness tracking device while performing physical activity such as running through a park. This activity data may modify an aspect of the virtual environment within the video game, such as increasing the stamina level of first user's in-game character. This may result in the first user's in-game character being able to run longer and/or faster. The video game may be played co-incidentally (e.g. substantially simultaneously) with the performance of the physical activity. Consequently, the activity data measured during the physical activity may be live-streamed to the information processing apparatus executing the video game. Alternatively, activity data may be transmitted to the information processing apparatus periodically, such as, say, after every 30, 40 or 50 seconds, or the like. Transmitting activity data to input unit 230, 330 while the user is currently performing physical activity not directly related to playing a video game shall be discussed later herein.

Turning now to Figure 4, in which parts 410-440 correspond to parts 310-340 of Figure 3 and 210-240 of Figure 2 except where stated otherwise, in embodiments of the present description, information processing system 600 comprises a first information processing apparatus 400 according to that which has been previously discussed herein, and a second information processing apparatus 500 comprising measuring unit 510 configured to measure activity data, and output unit 520 configured to transmit the activity data. Such embodiments enable activity data to be transferred from second information processing apparatus 500 to first information processing apparatus 400. It will be appreciated that information processing system 600 may also optionally comprise a generating unit and calibrating unit like information processing system 300.

In embodiments of the present description, second processing apparatus 500 may be a mobile phone (e.g. phone 500A of Figure 1), a fitness tracking device (e.g. tracker 500B of Figure 1), a portable video game console, or a periphery device in communication with any of the preceding. A fitness tracking device may be, say, a device which can be worn around, say, a user's wrist, and can be used to measure data associated with the physical activity being performed by the user, such as a heart rate, a number of calories burnt, or the like, for example. A periphery device may be, say, a handheld controller, a pair of earphones, a pair of headphones, a microphone, or the like, for example.

In embodiments of the present description, measuring unit 510 may be an accelerometer or other motion detecting means, a heart rate sensor, a GPS tracking unit or other location tracking means, a pedometer, a microphone, a camera, or any combination of the preceding, for example.

In embodiments of the present description, measuring unit 510 is configured to measure activity data. As previously mentioned, activity data is data associated with physical activity of a user who is not directly playing the video game. For example, in order to modify an aspect of the virtual environment within a video game, the user may be required to achieved a heart rate of, say, 140 bpm. Subsequently, the user wears, holds or otherwise affixes second information processing apparatus 500 to themselves and goes to perform a physical activity, such as, say, rowing on a rowing machine. Measuring unit 510 measures the user's activity data, that is, data associated with this physical activity. Such activity data may correspond to one or more aspects of a performance of this physical activity, such as a heart rate, a distance travelled, a location reached, a number of steps taken, a velocity, an acceleration, a breathing rate, a number of calories burned, and a detected motion of at least part of the user.

In embodiments of the present description, output unit 520 may be one or more data ports, such as USB ports, Ethernet ^{®} ports, WiFi ^{®} ports, Bluetooth ^{®} ports, or the like.

In embodiments of the present description, output unit 520 is configured to transmit the activity data. Such embodiments enable activity data to be transferred from second information processing apparatus 500 to first information processing apparatus 400. For example, after completing the physical activity, the user may return to their home (or more generally where the information processing system is set up for conventional use, or where its output is displayed to enable play). The user may then transmit activity data which has been measured during the performance of the physical activity from their, say, fitness tracking device to their, say, video game console. In this example, output unit 520 transmits the activity data to input unit 430. Once the video game console receives the activity data, one or more aspects of the virtual environment within a video game executed by the video game console may be modified, in dependence upon whether the received activity data satisfies one or more of the criteria. It will be appreciated that this transmission may be via an intermediate device such as a phone that has been previously paired or otherwise associated with the first and second information processing units; and/or via a server 1000 as described elsewhere herein.

In embodiments of the present description, output unit 520 may be configured to transmit a user ID. Such embodiments enable activity data to be associated with a given user. For example, before a user can use their, say, fitness tracking device, the user may be required to create a user account, which specifies which user is wearing the fitness tracking device. This user account can be used to associate activity data measured by the fitness tracking device with a given user, who may be identified using a user ID. Thus, the activity data may be associated with this user ID. This user ID may be, say, the user's name, a string of letters, numbers and/or symbols such as "USER#001", a name of a user account on the user's video game console such as "n00bslaya567", or the like. After the user has performed physical activity, the user may transmit activity data measured during the performance of the physical activity from their, say, fitness tracking device to their, say, video game console. Additionally, the fitness tracking device may transmit the user ID associated with the activity data to the video game console. In this example, output unit 520 transmits the activity data and the user ID to input unit 430. Once the video game console receives the activity data and the user ID, one or more aspects of the virtual environment within a video game executed by the video game console may be modified, in dependence upon whether the received activity data and the user ID satisfies one or more of the criteria. In this case, one of the criteria may be a required user ID, and the user ID provided by second information processing 520 may be required to match the required user ID in order for this criteria to be satisfied. Thus, one or more criteria associated with a video game may comprise a user ID associated with activity data.

Turning now to Figure 5, in embodiments of the present description, information processing system 1200 may comprise server 1000 comprising input unit 1010 configured to receive activity data and user ID, authentication unit 1020 configured to determine whether the user ID is associated with first information processing apparatus 700, and output unit 1030 configured to transmit the activity data in the event that the user ID is determined to be associated with first information processing apparatus 700. Such embodiments enable the transmission of activity data from second processing apparatus 900 (similar to second processing apparatus 500 except where stated otherwise) to first processing apparatus 700 (again similar to the first processing apparatuses of Figures 2, 3, and 4 except where stated otherwise) while the user is currently performing physical activity not directly related to playing a video game.

For example, a co-operative multi-player video game may require one or more users to provide game inputs via video game controllers, keyboards, mice or the like, and one or more other users to provide activity data by performing physical activity not directly related to the video game. Moreover, the video game may require physical activity to be performed co-incidentally with the playing of the video game. Consequently, in order to modify one or more aspects of the virtual environment within a video game, the video game may require activity data to be transmitted as a live stream from the user's fitness tracking device to the video game console executing the video game while the user performs the physical activity. As the fitness tracking device may be at too great a distance away from the video game console in order to directly transmit the activity data, server 900 may act as an intermediary that can receive the transmitted activity data from the fitness tracking device, and subsequently transmit the activity data to the video game console.

In embodiments of the present description, server 1000 may be any suitable server operating under suitable software instruction.

In embodiments of the present description, input unit 1010 may be one or more data ports, such as USB ports, Ethernet ^{®} ports, WiFi ^{®} ports, Bluetooth ^{®} ports, or the like.

In embodiments of the present description, input unit 1010 may be configured to receive an activity data and a user ID. Such embodiments enable activity data to be transferred from second information processing apparatus 900 to server 1000 while a user is currently performing physical activity not directly related to playing a video game. For example, the user may be wearing fitness tracking device while running through a park. The activity data associated with this physical activity and the user ID identifying the user may be transmitted from the fitness tracking device to a server via a network such as, say, the Internet. In this example, output unit 820 transmits the activity data and user ID to input unit 1010 via network 1100.

In embodiments of the present description, authentication unit 1020 may be one or more CPUs, one or more GPUs, or any combination of the preceding, for example.

In embodiments of the present description, authentication unit 1020 may be configured to determine whether the user ID is associated with first information processing apparatus 700. Such embodiments ensure that a user's activity data is to be transmitted to the user's video game console, for example. As previously mentioned, a user may have created a user account for their, say, fitness tracking device, for example. This user account can be used to associate activity data measured by the fitness tracking device with a user ID which identifies the user wearing the fitness tracking device while performing physical activity. After receiving the activity data and the user ID, the server performs an authentication process which involves determining whether the received user ID is associated with the user's video game console. For example, the received user ID may be the user's name, such as "John Birch". Subsequently, authentication unit 1020 may determine whether this user ID is associated with first processing apparatus 700 by determining whether the name "John Birch" corresponds to information associated with one or more user accounts on first information processing apparatus 700, for example. For example, in a successful authentication scenario, authentication unit 1010 has determined that the received user ID of "John Birch" corresponds to information associated with a user account on first information processing apparatus 700, the user account comprising username "JohnBirch007". In this scenario, the activity data received by server 1000 may be subsequently transmitted to information processing apparatus 700. Thus, determining whether a user ID is associated with first information processing apparatus 700 may comprise determining whether the user ID corresponds to information associated with one or more user accounts on first information processing apparatus 700.

It will be appreciated that where the game is being played co-incidentally with the performance of physical activity, the user account used with the first information processing apparatus may be that of another user currently providing conventional inputs to the game, and indeed the user involved in the fitness activity may not have an account associated with a first information processing apparatus. In this case, the user ID provided by the second information processing apparatus may be a session ID generated by the first information processing apparatus that is optionally only valid during the current play session, and the second information processing apparatus can send its data together with the session ID to the server. In this case the session ID may be sent by the first information processing apparatus to both the server and the second information processing apparatus by any suitable means, such as over the internet and via Bluetooth, respectively. The server can then associate the session ID with the first information processing apparatus in order to relay data received from the second information processing apparatus that is associated with the session ID. An app for the second information processing apparatus (or an associated communication device) may optionally be used to receive the session ID and subsequently send the data with it. The session ID may be any suitable ID, including a single use number, a friend account ID (if the user participating in the fitness activity is an identified friend within any administrative system serving the first information processing apparatus), or an email address (subject to verification by responding to an email sent to that address), or the like.

In embodiments of the present description, output unit 1030 may be one or more data ports, such as USB ports, Ethernet ^{®} ports, WiFi ^{®} ports, Bluetooth ^{®} ports, or the like.

In embodiments of the present description, output unit 1030 may be configured to transmit the activity data in the event that the user ID is determined to be associated with first information processing apparatus 700. Such embodiments enable activity data to be transferred from the server to the user's video game console. For example, once it has been determined that the received user ID of "John Birch" is associated with the user's video game console, the received activity data may be transmitted from the server to the user's video game console via a network such as, say, the Internet. In this example, output unit 1030 transmits the activity data to input unit 730 via network 1100.

In embodiments of the present description, output unit 1030 may be configured to transmit activity data as a live stream in the event that a video game is being played concurrently with the physical activity of a user, and transmit the activity data as either downloadable content or in a manner analogous to a live stream in the event that the video game is being played after the physical activity of the user. Such embodiments enable novel multi-player and single-player gaming experiences.

In the event that a video game is being played concurrently with the physical activity of a user, the video game may, for example, require one or more users to provide game inputs via video game controllers, keyboards, mice or the like, and one or more other users to provide activity data by performing physical activity not directly related to the video game. For example, the video game may require one or more users to provide game inputs which cause an in-game character to fire a weapon at a boss during a final boss fight. Moreover, this video game may require one or more users to concurrently provide activity data (such as velocity data) which satisfies certain criteria (such as reach a velocity of at least, say, 10 mph) in order to provide a supply of ammunition for the weapon being fired at the boss, wherein providing activity data may be the only way that supplies of ammunition are provided to the in-game character. In this example, the activity data may be live-streamed from the server to the video game console executing the video game. This live-streamed activity data may comprise time series data. This time series data may comprise data points, wherein each data point includes a timestamp denoting a particular point in time and the activity data measured at that point in time. These data points may be separated by time intervals whose duration is such that the time series data would be considered continuous in nature if plotted, such time intervals being, say, 0.003, 0.004, 0.005 seconds, or the like. Alternatively, the data points may be separated by time intervals whose duration is such that the time series data would be considered discrete in nature if plotted, such time intervals being, say, 0.5 seconds, 1 second, 10 seconds, 30 seconds, 1 minute, or the like. In this example, output unit 1030 transmits the activity data to input unit 730 as a live stream via network 1100. While receiving the live-streamed activity data (such as velocity data), the video game console may determine in real-time (that is, during the final boss fight) whether each of the data points of the live-streamed activity data satisfies the criteria (such as reach a velocity of at least, say, 10 mph). In this example, processing unit 740 determines whether a data point satisfies the criteria. Once the video game console has determined that a particular data point has satisfied the criteria, the video game console may then modify one or more aspects of the virtual environment within the video game in order to provide the in-game character with a supply of ammunition while the final boss fight is occurring.

In the event that a video game is being played after the physical activity, the video game may, for example, require a user to provide game inputs via video game controllers, keyboards, mice or the like, and provide activity data by performing physical activity not directly related to the video game. However, given that the user cannot play the video game at the same time as performing physical activity, the video game may require the user to perform physical activity before playing, say, a final boss fight. For example, the video game may require the user to provide activity data (such as velocity data) which satisfies certain criteria (such as velocity of at least, say, 10 mph) in order to provide their in-game character with a supply of ammunition for the in-game character's weapon, wherein providing activity data may be the only way that supplies of ammunition are provided to the in-game character. Subsequently the user wears their, say, fitness tracking device and goes running. The activity data measured during the run may be transmitted to a server via a network such as the Internet. When the user returns to their, say, video game console, the user may choose to transmit the measured activity data as downloadable content from the server to their video game console via a network such as the Internet. In this example, output unit 1030 transmits the activity data to input unit 730 as a live stream via network 1100. The video game console may subsequently determine whether any of the data points of the measured activity data (such as velocity data) satisfies the criteria (such as reach a velocity of at least, say, 10 mph) before the start of the final boss fight. In this example, processing unit 740 determines whether a data point satisfies the criteria. Once the video game console has determined that a particular data point has satisfied the criteria, the video game console may then modify one or more aspects of the virtual environment within the video game in order to provide the in-game character with a supply of ammunition before the start of the final boss fight.

Alternatively, the user may choose to transmit the measured activity data in a manner analogous to a live stream from the server to their video game console via a network such as the Internet. In this example, output unit 1030 transmits the activity data to input unit 730 as a live stream via network 1100. Once the user starts the final boss fight, the server may transmit the measured activity data to the video game console via a network in such a manner so as to emulate the way in which the measured activity data would be transmitted to the video game console if the measured activity data were being live-streamed from the server during the final boss fight, hereinafter called "quasi-live-streamed" activity data. While receiving the quasi-live-streamed activity data (such as velocity data), the video game console may determine in real-time (that is, during the final boss fight) whether each of the data points of the quasi-live-streamed activity data satisfies the criteria (such as reach a velocity of at least, say, 10 mph). In this example, processing unit 740 determines whether a data point satisfies the criteria. Once the video game console has determined that a particular data point has satisfied the criteria, the video game console may then modify one or more aspects of the virtual environment within the video game in order to provide the in-game character with a supply of ammunition while the final boss fight is occurring.

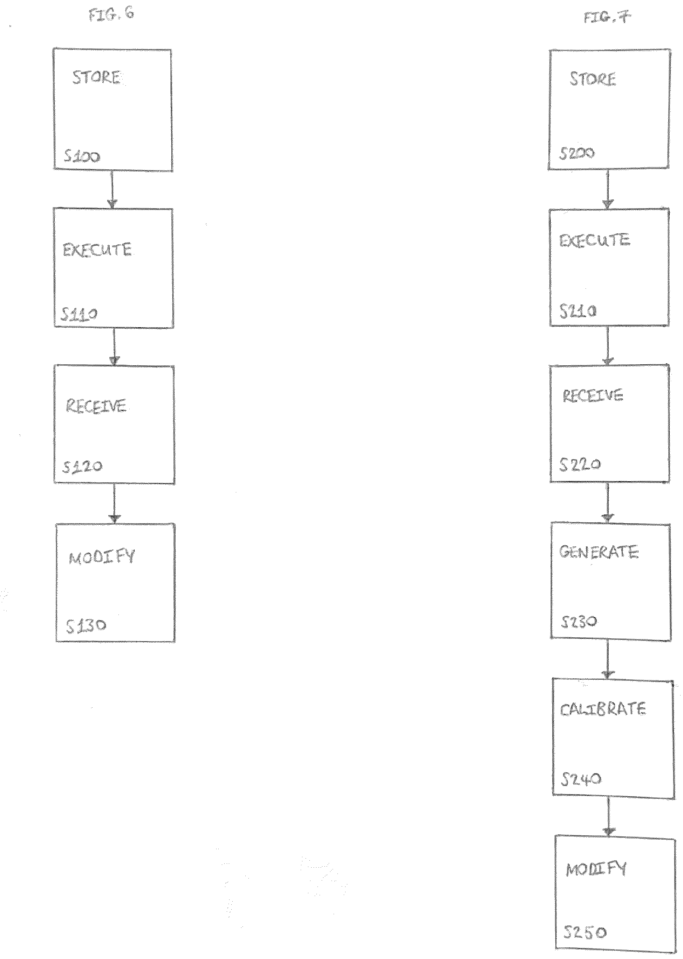

Turning now to Figure 6, an information processing method comprises the following steps:
Step S100: storing one or more criteria associated with a video game, as described elsewhere herein.
Step S 110: executing the video game, as described elsewhere herein.
Step S120: receiving activity data, wherein the activity data is data associated with physical activity of a user who is not directly playing the videogame, as described elsewhere herein.
Step S130: modifying one or more aspects of a virtual environment within the executed video game when the received activity data satisfies one or more of the criteria, as described elsewhere herein.

Turning now to Figure 7 where the named steps correspond to the named steps in Figure 6 unless otherwise stated, the information processing method may similarly comprise one or more of the following additional steps:
Step S230: generating, based on data associated with an environment in which physical activity is to be performed, one or more criteria associated with the video game, wherein receiving step S220 comprises receiving the data associated with the environment in which physical activity is to be performed.
Step S240: adjusting, based on data associated with a current fitness level of the user, one or more of the criteria associated with the video game, wherein receiving step S220 comprises receiving the data associated with a current fitness level of the user.

Moreover, the information processing method may be adapted in the following way:
Step S130, S250: may comprise modifying one or more aspects of the virtual environment within the video game based on one or more game inputs; and
Step S 120, S220: may comprise receiving the one or more game inputs, wherein the one or more game inputs are received from one or more of a video game controller, a keyboard, a mouse, a microphone, and a camera.

It will be apparent to a person skilled in the art that variations in the above method corresponding to operation of the various embodiments of the apparatus as described and claimed herein are considered within the scope of the present invention.

It will be appreciated that the above methods may be carried out on conventional hardware (such as entertainment system 10 and similar first processing devices 200, 300, 400, 700, and similarly second processing devices 500, 900 and server 1000) suitably adapted as applicable by software instruction or by the inclusion or substitution of dedicated hardware.

Thus the required adaptation to existing parts of a conventional equivalent device may be implemented in the form of a computer program product comprising processor implementable instructions stored on a non-transitory machine-readable medium such as a floppy disk, optical disk, hard disk, solid state disk, PROM, RAM, flash memory or any combination of these or other storage media, or realised in hardware as an ASIC (application specific integrated circuit) or an FPGA (field programmable gate array) or other configurable circuit suitable to use in adapting the conventional equivalent device. Separately, such a computer program may be transmitted via data signals on a network such as an Ethernet, a wireless network, the Internet, or any combination of these or other networks.

The foregoing discussion discloses and describes merely exemplary embodiments of the present invention. As will be understood by those skilled in the art, the present invention may be embodied in other specific forms without departing from the spirit or essential characteristics thereof. Accordingly, the disclosure of the present invention is intended to be illustrative, but not limiting of the scope of the invention, as well as other claims. The disclosure, including any readily discernible variants of the teachings herein, defines, in part, the scope of the foregoing claim terminology such that no inventive subject matter is dedicated to the public.

## Claims

1. An information processing apparatus, comprising:
a storage unit configured to store one or more criteria associated with a video game;
an executing unit configured to execute the video game;
an input unit configured to receive activity data, wherein the activity data is data associated with physical activity of a user who is not directly playing the video game; and
a processing unit configured to modify one or more aspects of a virtual environment within the executed video game when the received activity data satisfies one or more of the criteria.

2. An information processing apparatus according to claim 1, wherein the activity data is one or more selected from the list consisting of:
i. a heart rate;
ii. a distance travelled;
iii. a location reached;
iv. a number of steps taken;
v. a velocity;
vi. an acceleration;
vii. a breathing rate;
viii. a number of calories burned; and
ix. a detected motion of at least part of the user.

3. An information processing apparatus according to any preceding claim, comprising a generating unit configured to generate, based on data associated with an environment in which physical activity is to be performed, one or more criteria associated with the video game, wherein the input unit is configured to receive the data associated with the environment in which physical activity is to be performed.

4. An information processing apparatus according to any preceding claim, comprising a calibration unit configured to adjust, based on data associated with a current fitness level of the user, one or more of the criteria associated with the video game, wherein the input unit is configured to receive the data associated with a current fitness level of the user.

5. An information processing apparatus according to any preceding claim, wherein the processing unit is configured to modify one or more aspects of the virtual environment within the video game based on one or more game inputs, wherein the input unit is configured to receive the one or more game inputs, wherein the one or more game inputs are received from one or more selected from the list consisting of:
i. a video game controller;
ii. a keyboard;
iii. a mouse;
iv. a microphone; and
v. a camera.

6. An information processing system, comprising:
a first information processing apparatus according to any preceding claim; and
a second information processing apparatus, comprising:
a measuring unit configured to measure activity data; and
an output unit configured to transmit the activity data.

7. An information processing system according to claim 6, wherein the second information processing apparatus is one selected from the list consisting of:
i. a mobile phone;
ii. a fitness tracking device;
iii. a portable video game console; and
iv. a periphery device in communication with any of the preceding alternatives in this list.

8. An information processing system according to claim 6 or claim 7, wherein the output unit of the second information processing apparatus is configured to transmit a user ID.

9. An information processing system according to claim 8, comprising:
a server, comprising:
an input unit configured to receive the activity data and the user ID;
an authentication unit configured to determine whether the user ID is associated with the first information processing apparatus; and
an output unit configured to transmit the activity data in the event that the user ID is determined to be associated with the first information processing apparatus.

10. An information processing system according to claim 9, wherein the output unit of the server is configured to:
transmit the activity data as a live stream in the event that the video game is being played concurrently with the physical activity of the user; and
transmit the activity data as either downloadable content or in a manner analogous to a live stream in the event that the video game is being played after the physical activity of the user.

11. An information processing method, comprising:
storing one or more criteria associated with a video game;
executing the video game;
receiving activity data, wherein the activity data is data associated with physical activity of a user who is not directly playing the videogame; and
modifying one or more aspects of a virtual environment within the executed video game when the received activity data satisfies one or more of the criteria.

12. An information processing method according to claim 11, comprising:
generating, based on data associated with an environment in which physical activity is to be performed, one or more criteria associated with the video game;
wherein the receiving step comprises receiving the data associated with the environment in which physical activity is to be performed.

13. An information processing method according to claim 11 or claim 12, comprising:
adjusting, based on data associated with a current fitness level of the user, one or more of the criteria associated with the video game;
wherein the receiving step comprises receiving the data associated with a current fitness level of the user.

14. An information processing method according to any one of claims 11-13, wherein:
the modifying step comprises modifying one or more aspects of the virtual environment within the video game based on the one or more game inputs; and
the receiving step comprises receiving the one or more game inputs, wherein the one or more game inputs are received from one or more selected from the list consisting of:
i. a video game controller;
ii. a keyboard;
iii. a mouse;
iv. a microphone; and
v. a camera.

15. A computer program comprising computer executable instructions adapted to cause a computer system to perform any one of the methods of claims 11-14.
